# EUROPEAN PATENT APPLICATION

(11) **EP 4 477 155 A1**
(43) Date of publication of application: **18.12.2024**
(21) Application number: 23315246.1
(22) Date of filing: 14.06.2023
(51) Int. Cl.: A61B 17/00, A61B 17/04, A61M 25/01, A61M 25/09, A61B 34/30, A61B 90/00

(54) **MANIPULATING DEVICE**

(71) Applicant: Caranx Medical, 75013 Paris (FR)
(72) Inventor: The designation of the inventor has not yet been filed
(74) Representative: Hepp Wenger Ryffel AG

(57) **Abstract**

The present invention relates to a manipulating device, a closing tool, a system and a method for closing an opening in a wall of a vessel of a patient. The manipulating device (10) is for operating a closing tool for closing an opening (2) in a wall (3) of a vessel (4) of a patient. The closing tool (100) comprises at least a delivery sheath (102), a sealing element (103), in particular comprising an anchor (110a) and a plug (110b), before application arranged in the delivery sheath (102), and at least one filament (104) connected to the sealing element (103). The manipulating device (10) has a base (11) to be positioned relative to patient and a plurality of actuating units (12). The actuating units (12) at least include a delivery sheath actuation unit (14) mounted on the base (11) for moving the delivery sheath (102) relative to the base (11) in a longitudinal direction (L), in particular for inserting the delivery sheath (102) into the vessel (4), and a tube actuation unit (13) mounted on the base (11) for moving a tube (105), in particular a pusher tube (105), with respect to the base (11) and/or the delivery sheath actuation system (14) in a longitudinal direction (L).

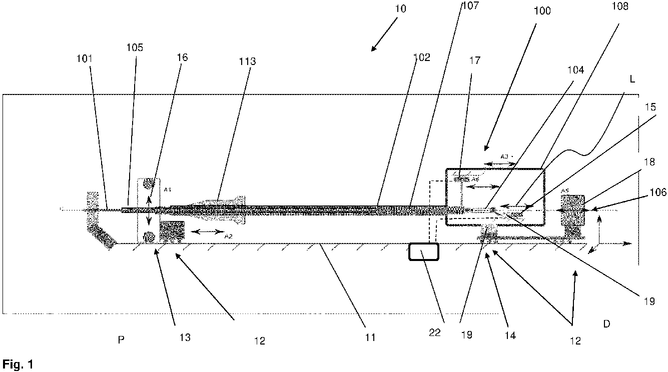

## Description

The present invention relates to a manipulating device, a closing tool, a system for closing an opening in a wall of a vessel and a method for closing an opening in a wall of a vessel of a patient.

Robotic surgery has been successfully used in medicine for vascular access. Percutaneous access of the vascular system for vascular device delivery is a common medical procedure. Typically, this involves using a hollow needle to puncture a vessel, then introducing an introducer sheath to open the puncture site for the introduction of catheters and guide wires for navigation through the vascular system to facilitate delivery. Robotic surgical tools may augment the surgeon's perception and control over surgical instruments for example by reducing tremor and scaling motions. Some systems have been extended to automate certain tasks.

US2015065916A1 discloses a robotic effector for moving a needle to puncture a vein under imaging.

US5415177A concerns an automatic guidewire placement device for an intravascular catheter and shows propelling means for an advancement of a guidewire through a needle into vessel.

US5749371A is related to an automatic guidewire placement device for medical catheters and discloses propelling means for advancement of a guidewire and a vacuum chamber for accelerating backflow of blood.

US2009118612A1 concerns ultrasound monitoring and post placement verification of a vascular access. https://arxiv.org/pdf/2107.02839.pdf is related to a robotically automated femoral vascular access.

Whereas the vascular access has been evaluated in various aspects, only a few systems are known for vascular closure. Once an intravascular procedure is completed, the procedure devices may be removed from the patient and pressure can be applied to the puncture site to stop the bleeding. Thereafter, the puncture may be sealed using a closure device. Closure devices are known which use a toggle (or anchor) member, a sealing member (or plug), and a filament (or suture). To lock the components together within the puncture, a locking member may be used.

The closing device may rely on local compression of two sealing elements, an anchor present on the interior and a plug present at an exterior vessel well. Most commonly, the internal anchoring feature is passed into and engaged inside the vessel, whereas the external plug is introduced on the exterior of the vessel while being at least partially aligned with the internal anchoring feature. Both features may be compressed against each other such as to cover and close the arterial opening.

US 10,555,727 B2 discloses a vascular closing device configured to be disposed along a guidewire towards a puncture of a vessel. The device provides tools which are manually operated.

However, as the closing device in a manually operated procedure is based on the surgeon's ability of perception and control.

It is the object of the present invention to overcome the drawbacks of the prior art. In particular, the manipulating device, the system and the method shall allow at least a (semi-) automated vascular closure of a percutaneous surgical entry.

These and other objects are solved with a manipulating device, a system and a method for closing an opening in a wall of a vessel according to the independent claims.

The manipulating device is for operating a closing tool for closing an opening in a wall of a vessel, preferably an artery.

The closing tool comprises at least a delivery sheath, a sealing element, arranged in the delivery sheath, and at least one filament connected to the sealing element. The sealing element in particular comprises an anchor, such as a toggle, and a plug, such as a collagen-patch.

The delivery sheath may be a static tube which may be connected to a device body. The static tube and the device body may form a device housing. The device body may be arranged at the distal part of the device housing, which may be arranged away from the patient.

The device housing may be connected to or envelop the distal parts of the concentrically arranged tubes of the closing tool.

Before use, the delivery sheath circumferentially envelopes at least a part of the sealing element and at least part of the tendons.

The delivery sheath may comprise scales on the outside for controlling the advancement into the patient. The delivery sheath should only be advanced until the distal end reaches a predetermined deployment depth.

When the plug is pressed against the anchor, and preferably when a peg lock maintains the position of anchor and plug, the sealing element is fixed.

The closing tool may be a generic patch-based closure device consisting of an instrument body, a multitude of concentrically arranged tubes, a pre-loaded collagen-patch, and an anchoring feature. The closing tool may be arrangeable along a guidewire. The closing tool may provide for a vascular closure by pressing an anchor on the inside of the vessel against a plug on the outside of the vessel to cover and close the arterial opening.

The manipulating device has a base to be positioned relative to patient and plurality of actuating units.

The actuating units may comprise rollers, for example pairs of rollers, wherein at least one roller is driven, belts, chains, a spindle and/or a motor unit. The actuation units may comprise a stepper motor. The actuating units may be suitable for causing an axial movement of tubes or filaments.

The actuation units may comprise connection parts, which connect the actuation unit to a respective tube, body, filament or other part to me moved.

The base may form a fixation frame and/or a housing of the manipulating device. The base may be connectable to a surgical robot or may be a part of a surgical robot.

The base may be positionable at an angle of 45° with respect to the skin surface of a patient.

The base may provide a reference for the deployment features of the closing tool; e.g. the moveable concentric tubes and anchoring features.

The actuating units may include a delivery sheath actuation unit mounted on the base for moving a delivery sheath relative to the base in a longitudinal direction, in particular for inserting the delivery sheath into the vessel and/or for retracting the delivery sheath from the vessel.

Within this application longitudinal means along the insertion direction, which typically is the direction of a guidewire leading out of the patient, which passes through the vessel and into the closing tool.

The actuating units include a delivery sheath actuation unit mounted on the base for moving the delivery tube. The delivery sheath actuation unit may provide for a movement of the delivery sheath with respect to base in a longitudinal direction.

The delivery sheath actuation unit may comprise a stepper motor.

The actuation unit may comprise a stepper motor providing a signal to be used by a control unit for controlling the insertion length.

The delivery sheath actuation unit may be connectable or connected to the delivery sheath.

The device housing, in particular the device body connected to the delivery sheath, may be connectable or connected to the access delivery sheath actuation unit.

The closing tool may comprise a tube actuation unit. The tube actuation unit allows for longitudinally translating a tube to and from the arterial opening along an already present path, for example created by an already-present guidewire.

The tube actuation unit may be suitable for longitudinally moving any of a multitude of concentrically arranged tubes of the closing tool, as for example for moving a pusher tube to be moved for pressing the plug on top of the vessel wall and optionally a peg log towards plug and the anchor.

Preferably the tube actuation unit is arranged at an end of the base which may form a proximal part of the base during use, such the delivery sheath actuation unit may be placed close to the patient, to provide a short transmission length.

The closing tool may be loadable to the manipulating device, such that the tubes and the filaments are connected to the actuating units.

The manipulating device may be used several times with new closing tools.

The manipulating device may comprise a receiving interface for loading the closing tool._The closing tool may be provided in form of a disposable cassette which may be inserted in the receiving interface of the manipulating device.

The actuating units may include a filament actuation unit for tensioning and releasing the filament(s), in particular for releasing the anchor, turning the anchor and/or for pulling the anchor to the inside to the vessel wall.

The filament actuation unit may comprise means for linear actuation of the filaments, e.g. a linear guide with a spindle drive and a motor.

The filament actuation unit may be combined with a force measuring device which engages with the filament.

Movement of the delivery sheath as well as tensioning and releasing the filaments may be machine guided and thus a remote operation or a fully automated procedure is possible.

Generally, this leads to a higher precision and to a better traceability than a manually guided operation.

The filament actuation unit may be fixed to the base, in particular may be connected to the delivery sheath actuation unit.

Alternatively, a filament actuation unit for tensioning and releasing the filaments may be part of the closing tool and may be connected to the delivery sheath, for example in or on the device housing, in particular in the device body.

The filament actuation unit may be connected directly or indirectly to the delivery sheath, in particular may be fixed to the device body.

Alternatively or additionally, the manipulation device may comprise a control unit for controlling a filament actuation unit for tensioning and releasing the filaments.

The control unit may be adapted to send control signals to the filament actuation unit.

The control unit may be adapted to receive signals of the force measuring device and to adapt control of the filament actuation unit.

When loading the closing tool into the manipulation device, a connection between the control unit arranged on the base and the filament actuation unit arranged on the closing tool may be established.

The manipulation device may comprise an interface for establishing a connection between the control unit and the filament actuation unit.

The connection may be an electrical, magnetic, mechanical, pneumatical or hydraulic connection. The interface may provide for a transfer of signals and/or forces.

The tube actuation unit may comprise a gripper unit, which may be mounted on the tube actuation unit.

The gripper unit provides for a releasable connection between the tube actuation unit and any other tube, such as the pusher tube.

The tube actuation unit with the gripper unit may provide for a combined movement of a pusher tube and a release tube at least partly arranged within the delivery sheath.

The gripper unit may be formed by an adjustable distance between two opposing rollers.

The tube actuation unit may comprise two rollers arranged to be moveable towards and away from each other.

The tube actuation unit may comprise two opposing friction wheels of which at least one wheel is driven by an actuator, and which are arranged moveably with respect to each other as to adopt to varying diameters.

Alternatively, the tube actuation unit may comprise two opposing belts, of which at least one belt drive is driven by an actuator, which can open and close.

The actuating units may further include a release actuating unit for deploying at least a part of the sealing element. Preferably, the release actuating unit may provide for longitudinally moving a release tube of the closing tool in order to deploy the anchor and to move it out of the delivery sheath.

The release tube may be arranged radially within the delivery sheath and the release actuating unit may provide for longitudinally moving a release tube relative to the delivery sheath.

The release actuating unit may be fixed to the base, in particular may be connected to the delivery sheath actuation unit.

Alternatively, the release actuation unit may be a part of the closing tool and may be connected to the delivery sheath, for example in or on the device housing, in particular in the device body.

Alternatively, or additionally, the manipulation device may comprise a control unit for controlling a release actuating unit for deploying at least a part of the sealing element.

The control unit may be adapted to send control signals to the release actuating unit.

When loading the closing tool into the manipulation device, a connection between the control unit arranged on the manipulation device, for example on the base, and the release actuating unit arranged on the closing tool, for example in the device body, may be established.

The manipulation device may comprise an interface for establishing a connection between the control unit and the release actuating unit.

The connection may be an electrical, magnetic, mechanical, pneumatical and/or hydraulic connection. The interface may provide for a transfer of signals and/or forces.

The control unit may be the same as the control unit described above.

The release actuating unit may be mechanically coupled to the filament actuation unit, such when the filament actuation unit is operated the release actuating unit may be operated, too.

The control unit may hence be adapted to send only a signal to the release actuating unit or to the filament actuation unit for inducing an action of both units.

The actuating units may include a guidewire actuation unit for moving a guidewire relative to the base and/or relative to the closing tool in longitudinal direction.

The guidewire actuation unit may allow for the guidewire to be advanced through the closing tool.

Generally, examples for wire actuation can be found in other industrial solutions which require a wire feed, including metal wire or tube production, TIG/MIG welding, filament 3D printers, EDM wire cutter feeder, or the like.

Within the medical domain, existing solutions present in robotic catheter drivers could be implemented. For instance, the guidewire actuation unit may comprise two opposing friction wheels of which at least one wheel is driven by an actuator.

The guidewire actuation unit may comprise grippers which can temporarily attach to the guidewire, combined with a translational actuation to advance/retract along the insertion direction. The guidewire actuation unit may comprise two opposing belts, of which at least one belt drive is driven by an actuator. The guidewire actuation unit may comprise a capstan-wheel drive.

The guidewire actuation unit may be mounted on the base. The guidewire actuation unit may be arranged on a distal side of the base. The guidewire actuation unit may allow for the guidewire to be pulled out the patient and the closing tool.

Typically, a guidewire is already present when the manipulating device approaches the patient. The distal end of an existing guidewire may be inserted in the closing tool at the side which is close to the patient.

Alternatively, the manipulating device may comprise a backloading unit for inserting a guidewire into the closing tool from the distal end, which may be necessary, in case a new guidewire has to be applied to the patient, when the delivery sheath has already been introduced into the vessel.

The backloading unit may also be suitable for insertion of the proximal end of a guidewire into the distal end of any hollow instrument lumen, e.g. a dilator, insertion sheath or catheter.

Suitable embodiments could be found in conventional "peg-in-hole" industrial automation solutions, as well as wire-cut alignment solutions.

For instance, a funnel or conical cavity may progressively narrow a lumen to allow a guidewire to be sufficiently axially aligned with a hollow instrument lumen, in this case the closing tool.

The manipulating device may include an insertion actuation unit for longitudinal movement of an insertion sheath.

The insertion actuation unit may be part of the actuating units and/or may be mounted on the base.

Alternatively, the insertion actuation unit may be mounted on a separate insertion base. The insertion base may be moveable with respect to the patient, and may be used to align the instrument insertion path with the surgical entry path. An alignment of the insertion base and the base can be done manually by a human operator, or with the aid of a positioning device such as a robotic arm.

For introducing the closing tool, an insertion sheath may be provided, which preferably has a tapering diameter along the guidewire. The narrow end may easily be guided though the vessel opening and the wider end may be suitable for receiving the delivery sheath and/or the access tube. The insertion sheath or the introducer may be inserted into the vessel by means of the insertion actuation unit.

The manipulating device may include an alignment tool, for longitudinally aligning the manipulating device and/or the closing tool loaded to the manipulating device with respect to an inser-tion sheath already placed at a patient.

For estimating the deployment depth of the delivery sheath, the manipulating device may rely on data that has been previously collected.

Alternatively, the manipulating device may comprise a measurement device for measuring the deployment depth. The measurement device may comprise an imaging device, such as an ultrasound tool. The distance of the vessel opening from the surface of the patient may be automatically determined by a control unit.

The measurement device may comprise a measurement gauge actuating unit for moving a measurement gauge with a measuring scale in the longitudinal direction.

The measurement gauge may be hollow and have a lateral opening at the distal end. The measurement gauge may be inserted into the vessel and may be slowly pulled out by means of the measurement gauge actuating unit. When blood stops escaping the lateral opening the proximal end of the measurement gauge has left the vessel. The distance between vessel opening and surface can be read out from the measuring scale, for example by an operator or by an imaging tool. The distance can be determined automatically, for example based on signals of a stepper motor of the measurement gauge actuating unit.

The manipulating device may comprise a cutting tool actuating unit for actuating a cutting tool for cutting the filament(s). In particular, the manipulating device may comprise a cutting tool, such as a knife.

After pressing the pressing the plug to the anchor, the filaments may be cut by means of a cutting tool which may be moved by means of the cutting tool actuating unit.

At least one of the actuation units may comprise a force sensing element for detecting axial forces acting on the delivery sheath and/or on the filament(s) or any other tube or wire actuated by the respective actuation unit.

The manipulating device may comprise a control unit for sending control signals to at least one of the actuation units, and in particular for receiving control signals from a computing unit of a, preferably autonomous or teleoperational, surgical system.

The control unit may be adapted to receive data from the force sensing element and may be adapted to send control signals for controlling and in particular limiting the movement of a tube or a wire in case the received data exceeds a predetermined range.

The force sensing elements may interact with the patient and may provide immediate feedback about the respective procedure step. Depending on the feedback received by the force sensing elements at least one step of the closing procedure may be interrupted, stopped, amended or repeated.

Forces transferred by the tubes on the tissue of the patient which are considered to be harmful may be avoided.

The control unit may be the same as the control unit mentioned above.

The manipulating device may be part of a surgical system for automated vessel closing.

A closing tool for use with a manipulating device according as described above, comprises at least a delivery sheath, a sealing element, preferably comprising an anchor and a plug arranged in the delivery sheath, and filaments connected to the sealing element. The closing tool comprises an interface for connecting the delivery sheath to a delivery sheath actuation unit of the manipulating device.

The closing tool may be provided as a disposable cartridge to be used with a re-usable manipulation device.

The closing tool may comprise a pusher tube and in particular an interface for connecting the pusher tube to the tube actuation system of the manipulating device.

The closing tool may comprise a filament actuation unit for tensioning and releasing the filaments and in particular an interface for connecting the filament actuation unit to a control unit of the manipulating device. Alternatively, the closing tool may comprise an interface for connecting the filaments to a filament actuation unit of the manipulating device.

The closing tool may comprise a release actuating unit for deploying at least a part of the sealing element preferably for longitudinally moving a release tube of the closing tool, and in particular an interface for connecting the release actuating unit to a control unit of the manipulating device. Alternatively, the closing tool may comprise an interface for connecting a release tube of the closing tool to a release actuating unit of the manipulating device.

The interfaces may provide for an electrical, magnetic, mechanical, pneumatical or hydraulic connection. The interfaces may provide for a transfer of signals and/or forces.

A system for closing an opening in a wall of a vessel comprises a manipulating device as described above and a closing tool, wherein the closing tool comprises at least a delivery sheath, preferably being connected to a device body and forming a housing, a sealing element, preferably comprising an anchor and a plug arranged in the delivery sheath, and at least one filament(s) connected to the sealing element. The closing tool may also comprise a peg lock.

The closing tool may further have a pusher tube to be guided within the delivery sheath for pushing the plug against the anchor and/or for pushing a peg lock against the sealing element.

The closing tool further optionally has a release tube for deploying at least a part of the sealing element and moving it out of the delivery sheath.

The system may comprise an insertion sheath.

The insertion sheath may have a connection interface at the distal end for connection with a proximal end of the delivery sheath.

A method for closing an opening in a wall of a vessel uses a manipulating device controlled by a control unit, preferably as described above, and comprises the stepper of loading a closing tool comprising at least a delivery sheath, a sealing element and filaments connected to the sealing element, to the manipulating device.

The method further comprises the automated steps of introducing a delivery sheath containing a sealing element, preferably comprising an anchor and a plug, through the opening into the vessel, of releasing the sealing element from the vessel, of retracting the delivery sheath and fixing the sealing element.

The method further may comprise the step of loading a closing tool comprising at least a delivery sheath, a sealing element and filaments connected to the sealing element, to the manipulating device.

Optionally the method comprises at least one of the following steps.

A base of the manipulation device may be placed with respect to the patient, preferably at an angle of 45° with respect to the skin surface of a patient, for example by a surgical robot.

An inserting sheath may be introduced into the vessel, which allows the delivery sheath, a release tube, a pusher tube and/or the sealing element to be introduced into the vessel, for example by means of an delivery sheath actuation unit.

A guidewire may be introduced into the vessel, into the manipulation device and/or into the closing tool, in particular by means of a guidewire actuation unit.

The minimal deployment depth and optionally the vessel diameter may be determined, in particular by a measurement with an image device. Alternatively, the minimal deployment depth and/or the vessel diameter may be read out from a storage by the control unit.

the deliver sheath may be coupled to the insertion sheath and/or introduced into the vessel. The deliver sheath may be advanced and/or retracted under the control unit according to the determined deployment depth.

The sealing element may be released by means of a release tube, which pushes at least the anchor out of the delivery sheath, in particular by means of the release actuation unit.

The delivery sheath and the release tube may be retracted from the patient, in particular by means of the delivery sheath actuation unit and/or the tube actuation unit.

By pulling the filament(s), in particular by means of the filament actuation unit, the anchor may be pressed against the inside of the vessel wall.

The plug, for example a collagen-patch may be pushed against the anchor from the outside of the vessel by a pusher tube, in particular by means of the tube actuation unit.

A peg lock may be pushed against the sealing element by the pusher tube. Plug and peg lock may be connected.

The guidewire may be retracted, preferably after fixing the sealing element.

The filaments may be cut close to the peg lock by a cutting tool, in particular by means of the cutting tool actuation unit.

Optionally, it may be tested if the closure is tight.

The system may comprise a local imaging device, such as an ultrasound probe for supervision to validate a tight closure. Validation can be tested by a local scan of the vessel. The absence of hematoma can be evaluated visually.

Alternatively, by use of ultrasound doppler imaging a flow decrease may be quantified.

Ultrasound imaging may be used both for initial insertion depth measurement at the start of the procedure for determining the anchor deployment depth as well as for validation of a tight closure.

Alternatively, a contrast shot can be taken using a catheter present in the artery, e.g. a radial pigtail. By fluoroscopy imaging a leak might be observed.

Preferably the local imaging device, preferable an ultrasound transducer, images the to-be-closed arterial opening, the steps of the closure procedure acted out upon it and the closed opening.

The local imaging device may also be used to measure the to-be-closed arterial opening area for setting up a detailed procedure plan, to real-time adjust the deployment depth, tube movements and/or the anchor positioning, and/or to monitor procedure steps for comparison with a procedure plan.

The invention also provides a computer program comprising a program code for carrying out the steps of the method as described above when the program is executed on a computer.

The invention also provides a computer program product which can be loaded directly into an internal memory of a digital computer and which comprises software code portions executing the method steps as described above when the program is running on a computer.

The invention is explained in the following by means of exemplary embodiments and the accompanying drawing, in which
- Figure 1: is a schematic presentation of a system for closing an opening in a wall of a vessel;
- Figure 2: is second schematic presentation of the system,
- Figure 3: is a third schematic presentation of the system.

Figures 1-3 show schematic presentation of a system 1 for closing an opening in a wall of a vessel. In figure 2 the system 1 is shown arranged with respect to the skin 5 of a patient. In figure 3 the system 1 is shown after placing the sealing element 3.

The system 1 for closing an opening 2 in a wall 3 of a vessel 4 (see figure 2) comprises a manipulating device 10 for operating a closing tool 100.

The closing tool 100 comprises a delivery sheath 102, and filaments 104 connected to the sealing element 103 (see figure 3) before use.

The delivery sheath 102 is connected to a device body 108 at the distal end D.

The delivery sheath 102 envelopes at least a part of a release tube 107 for deploying the sealing element out of the delivery sheath 102 and at least a part of a pusher tube 105 for pushing the plug 110b against the anchor 110a and eventually for pushing a peg lock against the plug 110b to fix the sealing element 103. The tubes 105, 107 and 103 are concentrically arranged.

The manipulating device 10 has a base 11 to be positioned relative to a patient and at plurality of actuating units 12.

The actuating units 12 include a delivery sheath actuation unit 14 mounted on the base 11 at a distal end D.

The delivery sheath actuation system 14 is connected to the device body 108 and thus indirectly to the delivery sheath 102. The delivery sheath actuation system 14 may push or retract the delivery sheath 102 independently of the other tubes 105, 107.

The actuating units 12 include a tube actuation unit 13 mounted on the base 11 at a proximal end P.

The delivery sheath actuation system 14 and the tube actuation unit 13 may longitudinally move the complete closing tool 100 and thus all tubular elements 103, 105, 107 as well as the sealing element 103.

The tube actuation unit 13 comprises a gripper unit 16 for gripping and moving tubes, in particular the pusher tube 105 for longitudinal movement.

The gripper unit 106 has a sufficient clearance for passage of the delivery sheath 102 and eventually the insertion sheath 112 (see figures 2 and 3).

The system 1 further includes a filament actuation unit 15 for tensioning and releasing the filaments 104.

By tensioning and releasing the filaments 104 the anchor may be turned from a stretched transport position, which it takes when it is inside the delivery sheath, to a sealing position and may be pulled against the vessel wall from the inside.

The system 1 further includes a release actuating unit 17. The release actuating unit 17 is mounted on the device body 108 which is connected to the delivery actuation system 14.

The release actuating unit 17 provides for a longitudinal movement of the release tube 107 in a range of 0.5-3cm, preferably 1-2cm.

The manipulation device 10 comprises a control unit 22 for controlling the actuating units 12, preferably according to a closing protocol and/or according to signals received from an autonomous or teleoperational surgical system.

The control unit 22 may be connected to the release actuating unit 17 and to the filament actuation unit 15.

The control unit may receive signals of force sensing elements 19, which detect axial forces acting on the delivery tube 102 and on the filaments 104. Depending on the signals the control unit 22 may amend the control of the actuating units 12.

The closing tool 100 may be loaded to the manipulation device 10, whereas the device body is connected to the delivery actuation system 14. The control unit 22 is connected to the release actuating unit 17 and to the filament actuation unit 15 and the tube actuation unit 13 is ready to receive and to transport a tube, for example the pusher tube 105.

After the sealing element 103 (see figure 3) has been placed, the closing tool 100 may be removed from the manipulation device 10 and, after sterilization the manipulation device 10 may be used again with a new closing tool 100.

The actuating units 12 include a guidewire actuation unit 18 for moving a guidewire 106 and a guidewire tube 101 relative to the base 11 and/or the closing tool 100 in longitudinal direction L. The guidewire actuation unit 18 may comprise a gripper unit to grip or release the guidewire 106 and the guidewire tube 101. After or during closing the opening 2 in the vessel 4 (see figure 2) the guidewire 106 maybe pulled out of the patient. The guidewire 106 may be arranged partly in a guidewire tube 101 and is redundant once the guidewire 105 has been inserted through the closing tool 100.

The guidewire actuation unit 18 is mounted on the delivery sheath actuation system 14, but may also be mounted directly on the base 11.

Typically, the guidewire 106 is already present at the patient, when the opening 2 in the vessel 4 (see figure 2) has to be closed.

The manipulation device 10 may comprise a backloading unit (not shown in the figures) to pass the system 1 over the guidewire 106. The backloading unit may also be separate from the system 1.

Before placing the sealing element 103 at the wall of the vessel 4, the pathlength from the surface of the patient to the vessel wall has to be determined. The pathlength can be obtained from a former measurement, for example a measurement during pre-op planning. The control unit of the system 1 may retrieve the path length from an electronic storage.

Alternatively, the measurement can be performed as a first part of the closing procedure. The pathlength measurement can be performed by a measuring stick inserted into the vessel by a measurement stick actuating unit not disclosed in the figure. The pathlength can be read from scales on the measuring gauge. The pathlength measurement can also be performed by means of an imaging device such as an ultrasound device.

Additionally, the maximal deployment depth, which corresponds to the diameter of the vessel can be determined.

Having determined the pathlength, the insertion of the closing tool 100 has to be prepared.

The closing tool 100 initially is filled with air. After loading to the manipulation device 10, all instrument lumens may be flushed with a sterile irrigating solution, for example a saline solution.

The manipulation device 10 may comprise pumping means and injection lines and (not shown in the figures) which may be interfaced with the tubes for providing the sterile solution.

The manipulating device 10 may include an insertion actuation unit (not shown in the figure) for longitudinal movement of an insertion sheath 112 (see figure 2), and in particular for longitudinal movement of a dilator (not shown in the figures). The dilator may be retracted and be cleared from the back end of the guidewire after placing the insertion sheath 112.

The insertion actuation unit may also be separate from the manipulating device 10.

The dilator and/or the insertion sheath 110 may be moved into the vessel, preferably along the guidewire 106.

Alternatively, the insertion sheath 112 may already be applied to the patient.

The system 1 may be passed over the guidewire 106 and may be brought in a proper position for the closing procedure.

The base 11 of the manipulation device 10 may be placed at an angle α of 45° with respect to the skin surface 5 of a patient.

Optionally, the insertion sheath 112 comprises an interfacing feature 109 for aligning and/or connecting the system 1 to an already-present large bore insertion sheath 112.

Once the insertion sheath 112 and the system 1 are connected and/or aligned, the closing tool 100 may be advanced to the entrance of the insertion sheath 112 by the delivery sheath actuation system 14.

The insertion sheath 112 and the system 1 may be retracted to match the target insertion depth.

The proximal end of the closing tool 100 can be inserted to or into the insertion sheath 112, the sealing element 103 may be introduced to be released at a set depth. The proximal end of the closing tool 100 may be inserted into the vessel 4, in particular the femoral artery, while the insertion forces are monitored by the force estimating elements 19 and a proper insertion force is applied. The insertion sheath 112 may be retracted (see figure 3).

The delivery sheath 102 hosting concentric tubes is moved into the artery 4 by the sheath actuation system 14.

The delivery sheath 102 may be gripped and translated at the same time by the access tube system 13.

If present, the guidewire tube 101 may be retracted, for example by the guidewire actuation unit 18.

A patch protection cover 113 is a temporary feature, and serves to pass the introducer port entry. It slides freely over the delivery sheath 102, which is statically fixed to the device body 108. It is too large to advance down towards the collagen plug 110b.

The anchor 110a is deployed inside the artery 4 in two steps: Deploying the anchor 110 b by pushing the anchor 110a forward by the release tube 107 and then angulating it using the filament actuation system 15 by tensioning the filaments 104.

Operating the filament actuation system 15 may release a filament tension against a preloaded spring to allow advancement of the release tube 107. Alternatively, the release actuating unit 17 may cause the release tube 107 to advance.

The delivery sheath 102 and the release tube 107as well as the insertion sheath 112 may be retracted, when the anchor 110a is engaged at the artery inner wall (see figure 3).

The insertion sheath 112 may actively be retracted using the tube actuation system 13, may be fixed to the device body 108, for example by a snap-fit connection, and may then be moved together with the closing tool 100 by the sheath actuation system 14.

While the device body 108 is retracted the anchor 110a is maintained under tension by retracting the filaments 104 by use of the filament actuating system 15.

The tube actuation system 13 and the gripper unit 16 may be used to grip the pusher tube 105 and to advance it towards the artery opening. The collagen plug 110b may be compressed onto the opening while holding the anchor 110 at a sustained force.

The pusher tube 105 is the inner most tube and needs to be gripped by the gripper unit 16 when exposed after retracting the delivery sheath 102. The pusher tube 105 may move freely between the collagen plug 110b (see figure 3) and the device body 108 across the guidewire 101 and the filaments 104. The pusher tube is arranged party concentric inside the release tube 107.

The pusher tube 105 may fix the sealing element 103 by pushing a peg lock (not shown in the figures) against the plug 110b. Then the pusher tube 105 may be retracted.

The peg lock may comprise locking means which provide for a connection with a filament, such that when the peg lock is fixed at his final destination, the plug maintains pressed onto the anchor.

The guidewire 106 may be retracted by the guidewire actuation unit 18.

The filaments 104 may be cut distally from the peg lock and the system 1 may by cleared from the patient.

Optionally, a cutting tool and a cutting tool actuation system (not shows in the figure) is present to clear the filaments 104 from the sealing element 103 after deployment.

This could be implemented at the filament actuation system 15 or at the gripper unit 16.

Alternatively, the cutting is done manually by a supervising operator or by a peripheral subsystem as part of a larger automatic surgical system.

Finally, it may be tested if the closure is tight, and the skin incision may be sutured.

## Claims

1. Manipulating device (10) for operating a closing tool for closing an opening (2) in a wall (3) of a vessel (4) of a patient,
the closing tool (100) comprising at least a delivery sheath (102), a sealing element (103), in particular comprising an anchor (110a) and a plug (110b), before application arranged in the delivery sheath (102), and at least one filament (104) connected to the sealing element (103),
wherein the manipulating device (10) has a base (11) to be positioned relative to patient and a plurality of actuating units (12),
the actuating units (12) including at least
- a delivery sheath actuation unit (14) mounted on the base (11) for moving the delivery sheath (102) relative to the base (11) in a longitudinal direction (L), in particular for inserting the delivery sheath (102) into the vessel (4) .
- a tube actuation unit (13) mounted on the base (11) for moving a tube (105), in particular a pusher tube (105), with respect to the base (11) and/or the delivery sheath actuation system (14) in a longitudinal direction (L).

2. Manipulating device according to claim 1, wherein
the actuating units (12) include a filament actuation unit (15) for tensioning and releasing the filaments (104)
or
the manipulation device comprises a control unit (22) for controlling a filament actuation unit (15) for tensioning and releasing the filaments (104) arranged on the closing tool (100).

3. Manipulating device according to claim 2, wherein the tube actuation unit (13) comprises a gripper unit (16).

4. Manipulating device according to one of the previous claims,
wherein
the actuating units (12) include a release actuating unit (17) for deploying at least a part of the sealing element (103), preferably for longitudinally moving a release tube (107) of the closing tool (100) in order to deploy at least a part of the sealing element (103), in particular the anchor (110a),
or
the manipulation device comprises a control unit (22) for controlling a release actuating unit (17) for deploying at least a part of the sealing element (103) arranged on the closing tool (100).

5. Manipulating device according to one of the previous claims,
wherein
the actuating units (12) include a guidewire actuation unit (18) for moving a guidewire (106) relative to the base (11) and/or the closing tool (100) in longitudinal direction (L) .

6. Manipulating device according to one of the previous claims,
wherein
the manipulating device (10) includes an insertion actuation unit for longitudinal movement of an insertion sheath (112) .

7. Manipulating device according to one of the previous claims,
wherein
the manipulating device (10) includes an alignment tool, for longitudinally aligning the manipulating device (10) with respect to an insertion sheath (112) already placed at a patient.

8. Manipulating device according to one of the previous claims,
wherein
the manipulating device (10) comprises a measurement device for measuring the deployment depth, in particular comprising a measurement gauge actuating unit for moving a measurement gauge with a measuring scale in the longitudinal direction (L) or an imaging device.

9. Manipulating device according to one of the previous claims, wherein the manipulating device (10) comprises a cutting tool actuating unit for actuating a cutting tool for cutting the filaments (104), in particular comprising a cutting tool, such as a knife.

10. Manipulating device according to one of the previous claims, wherein at least one of the actuation units (12) comprise a force sensing element (19) for detecting axial forces acting on the delivery sheath (102) and/or the filaments (104) .

11. Manipulating device according to one of the previous claims, wherein the manipulating device (10) comprises a control unit (22) for sending control signals to at least one of the actuation units (12), and in particular for receiving control signals from a computing unit of a, preferably autonomous or teleoperational, surgical system.

12. Closing tool (100) for use with a manipulating device according to one of the previous claims, the closing tool (100) comprising at least
- a delivery sheath (102),
- a sealing element (103), preferably comprising an anchor (110a) and a plug (110b) arranged in the delivery sheath (102),
- filaments (104) connected to the sealing element (103),
and
- an interface for connecting the delivery sheath (102) to a delivery sheath actuation unit (14) of the manipulating device;
the closing tool (100) further optionally comprising at least one of
- a pusher tube (105) to be guided within the delivery sheath (102) for pushing the sealing element (103),
- a release tube (107).

13. System (1) for closing an opening (2) in a wall (3) of a vessel (4) comprising a manipulating device (10) according to one of the claims 1-11, and comprising a closing tool (100), the closing tool (100) comprising at least
- a delivery sheath (102),
- a sealing element (103), preferably comprising an anchor (110a) and a plug (110b) arranged in the delivery sheath (102),
- filaments (104) connected to the sealing element (103),
wherein the closing tool (100) further optionally has at least one of
- a pusher tube (105) to be guided within the delivery sheath (102) for pushing the sealing element (103),
- a release tube (107).

14. System according to claim 12, wherein the system comprises an insertion sheath (112).

15. Method for closing an opening (2) in a wall (3) of a vessel (4), using a system (1) comprising a manipulating device (10) and a closing tool (100), preferably according to one of the claims 13 or 14, comprising the following steps:
- optionally loading a closing tool (100) comprising at least a delivery sheath (102), a sealing element (103) and filaments (104) connected to the sealing element (103), to the manipulating device,
- introducing the delivery sheath (102) through the opening (2) into the vessel (4),
- releasing the sealing element (103) from the delivery sheath (102),
- retracting the delivery sheath (102) and
- fixing the sealing element (103).

16. Method according to claim 15 comprising at least one of the following steps:
- Placing a base (11) of the manipulation device (10) with respect to the patient, preferably at an angle (α) of 45° with respect to the skin surface (5) of a patient;
- Introducing an inserting sheath (110) into the vessel (4), which allows the delivery sheath (102) to be introduced into the vessel (4);
- Introducing a guidewire (106) into the vessel (4) and/or into the closing tool (100);
- Measuring a minimal deployment depth and optionally the diameter of the vessel (4);
- Deploying at least a part of the sealing element (103) by longitudinally moving a release tube (107);
- Positioning the sealing element (103), in particular by tensioning and releasing the filaments (104);
- Retracting the delivery sheath (102) and the release tube (107) from the patient;
- Pulling the filaments (104), such that an anchor (110a) of the sealing element (103) may be pressed against the inside of the vessel wall (3);
- Pushing a plug (110b) of the sealing element (103), for example a collagen-patch, against an anchor (110a) of the sealing element (103) from the outside of the vessel (4), preferably by a pusher tube (105);
- Pushing a peg log (111) against the sealing element (103), preferably by a pusher tube (105), preferably connecting the plug (110b) and the peg lock (111);
- Retracting the guidewire (106), preferably after fixing the sealing element (103);
- Cutting filaments (104) close the sealing element (103), in particular close to the peg lock (111);
- Testing if closure is tight.

17. A computer program comprising a program code for carrying out the steps of the method according to any one of claims 15 to 16 when the program is executed on a computer.

18. A computer program product which can be loaded directly into an internal memory of a digital computer and which comprises software code portions executing the method steps of at least one of the claims 15 to 16 when the program is running on a computer.
